(19) Europäisches Patentamt
European Patent Office
Office européen des brevets

(11) **EP 2 053 914 B1**

(12) **EUROPEAN PATENT SPECIFICATION**

(45) Date of publication and mention
of the grant of the patent:
**26.01.2011 Bulletin 2011/04**

(21) Application number: **07801400.8**

(22) Date of filing: **07.08.2007**

(51) Int Cl.:
*A01K 67/02* (2006.01)    *G01N 33/04* (2006.01)

(86) International application number:
**PCT/EE2007/000014**

(87) International publication number:
**WO 2008/017311 (14.02.2008 Gazette 2008/07)**

(54) **METHOD OF IDENTIFYING OF COWS, SIRES AND BULL DAMS PRODUCING MILK WITH MODIFIED COAGULATION PROPERTIES**

VERFAHREN ZUR IDENTIFIZIERUNG VON KÜHEN, ZUCHTBULLEN UND ZUCHTSTIERMUTTERN DIE MILCH MIT MODIFIZIERTEN COAGULATIONSEIGENSCHAFTEN ERZEUGEN

PROCÉDÉ D'IDENTIFICATION DE VACHES, TAUREAUX ET MÈRES DE TAUREAUX PRODUISANT DU LAIT AYANT DES PROPRIÉTÉS DE COAGULATION MODIFIÉES.

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **10.08.2006 EE 200600029**

(43) Date of publication of application:
**06.05.2009 Bulletin 2009/19**

(73) Proprietor: **Oü Tervisliku Piima Biotehnoloogiate Arenduskeskus**
**51014 Tartu (EE)**

(72) Inventors:
• **PÄRNA, Elli**
  **EE51005 Tartu (EE)**
• **VALLAS, Mirjam**
  **EE54011 Tartu (EE)**
• **PÄRNA, Kalev**
  **EE51005 Tartu (EE)**
• **KAART, Tanel**
  **EE50106 Tartu (EE)**

(74) Representative: **Kahu, Sirje**
**OÜ Ustervall**
**Kivi 21-6**
**EE-51009 Tartu (EE)**

(56) References cited:
**WO-A-94/19934    WO-A-03/007705**

• **DATABASE BIOSIS [Online] BIOSCIENCES INFORMATION SERVICE, PHILADELPHIA, PA, US; January 1999 (1999-01), IKONEN T ET AL: "Genetic parameters for the milk coagulation properties and prevalence of noncoagulating milk in Finnish dairy cows" XP002460027 Database accession no. PREV199900126472 cited in the application & JOURNAL OF DAIRY SCIENCE, vol. 82, no. 1, January 1999 (1999-01), pages 205-214, ISSN: 0022-0302**
• **DATABASE BIOSIS [Online] BIOSCIENCES INFORMATION SERVICE, PHILADELPHIA, PA, US; 1992, OLOFFS K ET AL: "THE RELEVANCE OF PROTEIN VARIANTS TO GENETIC DIFFERENCES IN CHEESE MAKING PROPERTIES IN MILK" XP002460097 Database accession no. PREV199294016494 & ZUECHTUNGSKUNDE, vol. 64, no. 1, 1992, pages 20-26, ISSN: 0044-5401**

**Description**

TECHNICAL FIELD OF THE INVENTION

The invention relates to agriculture and methods of identifying of cans, rion and dull dans, producing milk with desirable coagulation properties.

BACKGROUND OF THE INVENTION

Enzymatic coagulation of milk is a process resulting of formation of curd after the addition of clotting enzyme to raw milk. The milk clotting ability determines the proportion of the milk that is converted to cheese. The milk, which is used for cheese production, should have good coagulation properties after the addition of clotting enzyme (chymosin). Milk coagulation parameters have a clear effect on cheese-making properties (FitzGerald, The Dairy Products Research Centre, Moorepark, DPRC No.19, 1999). The milk that aggregates and forms curd soon after the addition of the clotting enzyme is expected to produce higher dry matter cheese yields than the milk with poor coagulation properties does (Ikonen T. et al., J. Dairy Sci. 87(2), 458-467, 2004; FitzGerald, The Dairy Products Research Centre, Moorepark, DPRC No.19, 1999).

In Estonia, 8% of cows have at least one noncoagulating milk during lactation (Kubarsepp et al. Proc. 22th Nordic Assoc. Agric. Sci. Congr., Turku, Finland, p.90, 2003). In Finland, 13% of Finnish Ayrshire cows have noncoagulating milk (Tyrisevä et al., Dairy Sci. 87(11), 3958-3966, 2004).

The milk coagulation properties of individual cows are different. Genetic improvement of milk coagulation properties would potentially be well-supported means to improve the efficiency of cheese production (Ikonen T. Academic Diss., University of Helsinki, Department of Animal Science, Publications No. 49, 2000; Ikonen T. et al., J. Dairy Sci. 87 (2), 458-467, 2004). Genetic parameters of raw milk coagulation have been estimated only in a few studies and the results obtained are often not reliable because of small dataset or because of no utilisation of important information about relationship between animals. The basic conditions for genetic improvement of milk coagulation properties are good because much variation exists in these properties and 30 to 40% of this variation is additive genetic (Ikonen T. et al. Agric. Food Sci. Finl. 6:283-294,1997; J. Dairy Sci. 82:205-214, 1999; J. Dairy Sci. 87(2):458-467, 2004). A method of identification of cans, being capable of producing mien with desirable chariactedistics (desirably modified fart or cholesterol) has been described in WO03/007705 copin 2005.

DISCLOSURE OF THE INVENTION

The subject of the invention is a method of identifying individual cows and bull dams that produce milk with modified coagulation properties and sires whose daughters are expected to produce milk with modified coagulation properties. According to the disclosed method, milk curd firmness and coagulation time of cows, bull dams and daughters of sires are measured at least 3 times during the whole lactation and Legendre polynomials are used to estimate the level of these traits within the whole lactation, resulting in individual coagulation curves of curd firmness and coagulation time whose average levels are compared with reference levels to identify cows, bull dams and sires with milk coagulation properties. Cows and bull dams who produce milk with modified coagulation properties during the whole lactation and sires whose daughters are expected to produce milk with modified coagulation properties during the whole lactation are identified if the average milk curd firmness of cows is above 34.75 mm and average coagulation time is below 7.2 min, the average milk curd firmness of bull dams is above 36 mm and average coagulation time is below 6.6 min and the average milk curd firmness of daughters of said sires is above 29.5 mm and coagulation time is below 8.5 min.

The said method further comprises determination of κ-casein genotype of cows, bull dams and daughters of sires and identification that the average curd firmness bigger than E30>30.43 corresponds to the κ-casein genotype BB and the average curd firmness less than E30<26.07 corresponds to κ-casein genotypes EE and AE.

The method further comprises identification that the cows and bull dams and sires whose daughters are expected to produce milk with modified coagulation properties during the whole lactation are not bearing κ-casein E-allele.

Definitions.

Milk coagulation - enzymatic clotting of milk is a process resulting in formation of curd after addition clotting enzyme to raw milk.

Milk with modified coagulation properties - average curd firmness of milk of all cows is above 34.75 mm and average coagulation time of milk of all cows is less than 7.2 min. Sires with modified milk coagulation - average curd firmness of milk of their daughters is above 29.5 mm and average coagulation time of milk is less than 8.5 min.

Coagulation or enzymatic clotting is a process resulting in formation of curd after addition of clotting enzyme to milk. Enzymatic coagulation of milk is a process of three overlapping steps (Brown, R.J. and Ernstrom C.A., Milk clotting enzymes and cheese chemistry. Part I. Fundamentals of Dairy Chemistry. 3rd ed., N. P. Wong, R. Jenness, M. Keeney, and E. H. Marth, eds. Van Nostrand Reinhold, New York, 609-633, 1988). During the primary, enzymatic phase, chymosin, which is the clotting enzyme, splits κ-casein at the $Phe_{105}$-$Met_{106}$ bond into para-κ-casein and macropeptide. Because of this splitting of κ-casein, it begins to aggregate (the second phase of milk coagulation). Curd-firming time (R) describes the time between addition of clotting enzyme until the begginning of curd formation. During the third step of milk coagulation, aggregated casein micelles form a more or less firm gel structure. Curd firmness ($E_{30}$) describes the firmness of the curd 30 min after addition of the clotting enzyme. It is important for cheese production that milk coagulates quickly and forms firm curd after the addition of clotting enzyme (short clotting time and high level of curd firmness are desirable).

Milk coagulation varies during lactation, depending on many factors (like the stage of lactation, protein and calcium content of milk, pH, individuality of cow, somatic cell count, body condition score, breed, feeding, season).

Because of this, in order to estimate milk coagulation properties, in this invention the coagulation curve describing milk coagulation level during a long period - the whole lactation - is used instead of single measurements used so far. Based on the coagulation curve it is possible to find out the average milk coagulation level of the cow for any specific lactation day or period.

The invention provides new methods to estimate milk coagulation of individual cattle (cows and sires - based on their daughters) during the whole lactation and opportunities of generating selected dairy cattle populations, which can be used for production of milk with desirable coagulation properties.

For each cow having at least 3 observations in the data, a coagulation curve is fitted that is based on its milk coagulation properties, which characterizes the whole lactation of the individual cow.

For doing that, a regression model with random coefficients is used:

$$y_{ijk} = H_k + \sum_{n=1}^{3} b_n P_n(t) + \sum_{n=0}^{3} c_{in} P_n(t) + \varepsilon_{ijk}$$

where

$y_{ijk}$ - $j$-th value of $E_{30}$ or log(R) for cow $i$ of farm $k$  $H_k$ - fixed effect of farm $k$

$P_n(t)$ - value of Legendre polynomial of order $n$ at standardized milking day $t$

$b_n$ ja $c_{in}$ - coefficients of the model, calculated on basis of data

$\varepsilon_{ijk}$ - random error.

In addition to the farm effect the model with random regression coefficients contains the 3rd order Legendre polynomial, which is included as fixed effect factor but also as random effect factor, in order to be able to estimate individual curve of each cow, different from the average curve. Legendre polynomials are known for their flexibility in modelling observed data points. In addition, the process of calculation of estimates converges faster when using Legendre polynomials.

The inventors discovered that certain individual cows produce milk with desirable milk coagulation properties, characterised by relatively firm curd and relatively short clotting time. For example, 7.5% of Estonian cows produce milk with such properties (see example 1).

Modified milk specified by this method is characterised with average firmness of curd above 34.75 mm and average coagulation time less than 7.2 min (reference value of cows) and average curd firmness above 36 mm and coagulation time less than 6.6 min (reference value of bull dams).

Based on coagulation curves of daughters, curves are estimated also for sires, having in a trial at least 10 daughters. Coagulation curve relevant to bulls is calculated as an average curve of his daughters.

In order to compare bulls, one needs an index as a basis for ordering the bulls. Such an index is defined as the average value of coagulation trait calculated from summarized value of caoagulation trait over the whole lactation period, or, shortly, as the average level of coagulation curve relevant to the bull. The same summarized value was also used for preliminary estimation of raw milk coagulation properties heritability.

This is determined by using the present invention that daughters of sires are having average curd firmness above 29.5 mm and average milk coagulation time less 8.5 min for daughters' one lactation day (reference value of sires).

Based on the method described in this invention, it is disclosed that certain individual cows produce milk during the whole lactation with considerably better coagulation properties than the others (Table 3).

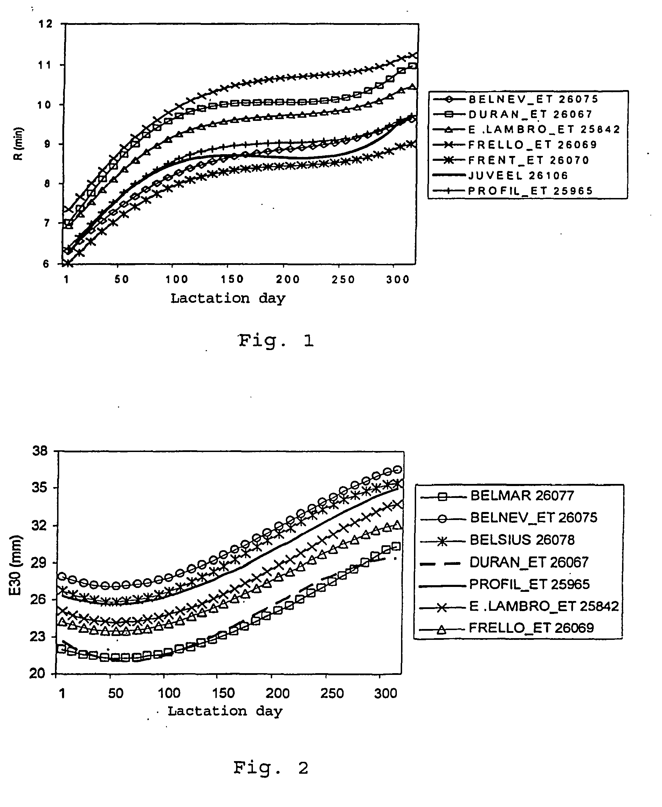

Based on the method of this invention, it is disclosed that daughters of certain sires produce milk during the whole lactation with considerably better coagulation properties than the others (Fig. 1, 2, 3, 4).

Based on the method of this invention it was possible to reveal that the coefficient of heritability $h^2$ of milk coagulation properties ($h^2$=0.55 for coagulation time, $h^2$=0.73 for curd firmness) is high, providing prerequisites for the genetic selection based on the traits above (Table 1).

The present invention is applicable for any variety of cattle breeds and/or crossbreeds known in the art. Methods known to those skilled in the art for estimation of breeding value of cattle for milk coagulation properties are used.

**Estimation of the whole lactation milk coagulation traits of individual cows comprises the following steps, where:**

- At least 3 milk samples from every individual cow were collected during lactation;
- Milk coagulation time (R) and curd firmness ($E_{30}$) were determined;
- Records for $E_{30}$ and logarithmic values of R were analysed by regression model with random coefficients;
- Legendre polynomials were used to model curd firmness and milk coagulation time of daughters of sires during the whole lactation;
- Using the model with random regression coefficients, for each cow an $E_{30}$ coagulation curve and an R coagulation curve were obtained, each individual curve being a 3rd order polynomial with coefficients depending on the cow;
- On the basis of coagulation curves numerical values of coagulation properties were found.

**Genetic evaluation of sires on the basis of their daughters' whole lactation milk coagulation comprises the following steps, where:**

- for daughters of each sire their milk coagulation time (R) and curd firmness ($E_{30}$) were determined at least 3 times during lactation;
- records for $E_{30}$ and logarithmic values of R were analysed by regression model with random coefficients;
- Legendre polynomials were used to model curd firmness and milk coagulation time of daughters of sires during the whole lactation;
- Using the model with random regression coefficients, for each cow an $E_{30}$ coagulation curve and a R coagulation curve were obtained, each individual curve being a 3rd order polynomial with coefficients depending on the cow;
- coagulation curves of sires were defined as averages of coagulation curves of their respective daughters;
- sires were compared by using ANOVA method, applied to aggregated values of $E_{30}$ and R of their respective daughters.

The present invention is applicable for any of variety of cattle breeds and/or crossbreeds known in the art. Well-known standard methods for the estimation of breeding value of cattle for milk coagulation properties are used.

**Identification of the cows producing milk with modified coagulation properties.**

To evaluate whether an individual cow produces milk with modified coagulation properties, curd firmness and coagulation time is determined at least 3 times during lactation. Any known method for this is suitable. Milk sample is collected from every individual cow separately. Methods for collecting milk samples are well known. The milk sample is fresh, not frozen. Determination of milk coagulation properties is carried out with well-known methods in the art and curd firmness, coagulation time and pH of milk are recorded.

Methods for determining curd firmness, coagulation time and pH and modificating obtained results from optical signal to millimeters are described in the paper (Pärna, E. et al., Genetic Improvement of Milk Coagulation Properties. Proc. Of the 8th World Congress of Genetics Applied to Livestock Production. Belo Horizonte, Brazil: Published by Organizing committee of the 8th World Congress of Genetics Applied to Livestock Production, 2006).

The above-mentioned results are used for the estimation of milk coagulation properties of individual cows, using Legendre polynomials. In this way an individual coagulation curve is obtained for curd firmness as well as for coagulation time for every single cow.

In order to decide whether an individual cow can produce milk with modified coagulation properties, the average level of curd firmness and coagulation time of its whole lactation, found from respective coagulation curves that are obtained by using Legendre polynomials, is compared with corresponding reference values.

Reference values of milk with modified coagulation properties provided by the invention are 34.75 mm and 7.2 min for curd firmness and coagulation time respectively. If the individual values exceed (curd firmness) or are less (coagulation time) than reference values, such cow is referred to as the cow producing milk with modified coagulation properties, or shortly - "a cheese cow".

The invention provides method of identification of bull dams if the average curd firmness during the whole lactation exceeds 36 mm and the average coagulation time is less 6.6 min. To estimate breeding value of cows well known art is used.

The invention provides a method to identify bull dams with modified coagulation properties, by using reference values of bull dams, and to generate progeny from those bull dams, also producing milk with modified milk coagulation properties. The method comprises milk sample collection during the whole lactation from potential bull dams at least 3 times, determination of coagulation properties and evaluation of coagulation curves. Those bull dams who by comparing with reference values are estimated to be bull dams with modified milk coagulation properties, are inseminated with sires who are considered to be sires with modified milk coagulation properties on the basis of their daughters' milk coagulation, or with young bulls whose breeding value is not estimated and thereafter milk coagulation properties of their progeny is also estimated. Usually progeny with desired properties is separated from other cows, resulting in increase of proportion of dairy cows with modified milk coagulation properties.

Standard cattle breeding methods used in the implementation of the invention are well known and widely used in the art (See e.g. Falconer & Mackay, Introduction to Quantitative Genetics, fourth edition. Longman, Essex, UK, 1996; Kinghorn et al., Use of New Technologies. Twynam Press, 2001). All daughters of sires of high genetic merit, whose semen is widely used, are tested for their milk coagulation properties. As the heritability of coagulation properties is high, it is recommended in the breeding of cattle to use sires who have many daughters producing milk with modified coagulation properties, by inseminating with their semen those cows who produce milk with modified coagulation properties. This is the way to increase the proportion of cows having modified coagulation properties of milk.

**Methods of estimation of sires with modified coagulation properties.**

The invention provides a method for estimation of sires with modified coagulation properties based on the whole lactation evaluation of their daughters' coagulation properties. The method comprises estimation of milk coagulation properties of the daughters of sires using methods described above during the whole lactation, evaluation of coagulation curves and estimation of sires based on the coagulation curves of their respective daughters. Sires' coagulation values are compared with the reference value and sires whose value exceeds the reference value are considered to be sires with modified milk coagulation properties. Breeding value estimation of sires' coagulation properties is based on well known in the art methods.

**Identification of genotype of cows, bull dams and based on daughters' evaluation of sires, producing during the whole lactation milk with an average curd firmness greater than $E_{30}>30.43$ and less than $E_{30}<26.07$.**

The invention provides a method for the genetic evaluation of the whole lactation milk coagulation properties of cattle using genetic markers. Identification of such genetic markers provide an opportunity to carry out marker assisted selection, making possible the identification of cattle as carriers of desirable or not desirable markers. In such case there is no need to evaluate sires based on their daughters performance. There is also an opportunity to superovulate carriers of desirable markers or not to use carriers of undesirable markers in breeding practice as bull dams or sires.

There is no opportunity of identifying cattle producing milk with modified coagulation properties on the basis of κ-casein genotype because different genotypes of κ-casein did not exceed reference values of modified milk coagulation properties, but still there is an opportunity to use κ-casein genotype as additional marker for the identification of cows producing milk with coagulation properties being in average better ($E_{30}>30.43$; BB-genotype) or in average worse ($E_{30}<26.07$; EE- and AE-genotype).

The method involves in general:

1)identification of cows producing milk with modified and non-modified coagulation properties,
2)obtaining DNA of cattle,
3)assaying polymorphism of the sample.

Relationship of κ-casein polymorphism with milk coagulation properties is well known in the art. Present invention provides an opportunity to use κ-casein genotype as a marker for evaluation of whole lactation coagulation properties.

A standard methodology for identification of relationship between polymorphism and any trait is known.

The present methodology involves:

1)evaluation of milk coagulation properties of cows producing milk with modified as well as with non-modified coagulation properties, using the above-mentioned methods,
2) obtaining DNA of cattle,
3)assay of κ-casein genotype using standard methods (Sabre D., Molekulaargeneetilise informatsiooni kasutamise võimalusi tõuaretuses. - Väitekiri põllumajandusteaduste magistrikraadi taotlemiseks loomakasvatuse erialal. Tartu, 83 lk., 2003).

All statistical analysis was done by using the SAS System® (SAS, Cary, NC, USA) (Example 3). For the identification of genotype it is possible to use cow, bull or calf at any stage of reproduction.

The invention provides an opportunity to estimate whole lactation milk coagulation properties of cows and breeding bulls (based on estimation of their daughters) according to the coagulation curve.

In order to estimate milk coagulation properties, in the present invention the coagulation curve describing milk coagulation level during a long period - the whole lactation - is used instead of single measurements used so far.

Based on the coagulation curve it is possible to find out the average milk coagulation level of the cow for any specific lactation day or period.

The invention provides an opportunity to generate a cow population (either physical or on info carrier) producing milk with modified coagulation properties, wherein milk of all cows has desirable coagulation properties, independently of breed of cattle.

The invention provides an opportunity to identify potential bull dams, producing milk with modified coagulation properties.

The invention provides an opportunity to identify sires having daughters producing milk with desirable coagulation properties.

The invention provides an opportunity to generate progeny, producing milk with desirable coagulation properties.

BRIEF DESCRIPTION OF THE DRAWINGS

Fig. 1 Milk coagulation time (R) during the lactation: averaged over daughters of 7 sires.
Fig. 2 Milk curd firmness ($E_{30}$) during the lactation: averaged over daughters of 7 sires.
Fig. 3 Area ($S\_E_{30}$) below predicted lactation curve of milk curd firmness ($E_{30}$): averages by bulls.
Fig. 4 Area ($S\_R$) below predicted lactation curve of milk coagulation time (R): averages by bulls.
Fig. 5 Fractions of κ-casein genotypes in Estonian Holstein cows population.
Fig. 6 Lactation curves of milk curd firmness ($E_{30}$) by κ-casein genotypes.
Fig. 7 Lactation curves of milk coagulation time (R) by κ-casein genotypes.

DESCRIPTION OF EMBODIMENTS

Cows, bull dams and sires based on their daughters, producing milk with modified coagulation properties were identified according to the methods, which are the subjects of this invention. Cows, bull dams and sires based on their daughters, producing milk with curd firmness average above $E_{30}>30.43$ and less than $E_{30}<26.07$ were identified. Thereafter the κ-casein genotype of bull dams and daughters of sires was determined and the relationship between κ-casein genotype and the whole lactation milk coagulation properties was established.

EXAMPLE 1

Determination of the milk coagulation properties (curd firmness and coagulation time) and estimation of individual cows based on whole lactation coagulation properties of milk.

Based on the milk coagulation properties of individual cows, a coagulation curve for every single cow was constructed, which was used for the estimation of all lactatation milk coagulation properties of the cow and this way the cows with the best coagulation properties were identified.

Procedure of the trial. 1) Determination of milk coagulation properties

Milk coagulation properties of every cow under the evaluation were determined at least 3 times during the lactation. Prior to the assessment of rennet coagulation properties, the samples were tempered to the renneting temperature. (35˚C). The rennet (Milase MRS 750 IMCU/ml; CSK Food Enrichment B.V., The Netherlands) used in analyses was diluted 1:100 (v/v) with distilled water and 0.2 ml of the solution was added to 10 ml milk. Milk coagulation parameters (rennet coagulation time (R), firmness of curd ($E_{30}$)) were determined using the Optigraph (Ysebaert, Frepillon, France, Optigraph system recipes were: R slope = 1.784 and R offset = -2.303) at 35˚C. To determine the firmness of the curd, the Optigraph signal was used 30 minutes after rennet addition. Electrical parameters obtained by Optigraph were converted into millimeters (Pärna, E. et al., Genetic Improvement of Milk Coagulation Properties. Proc. Of the 8th World Congress of Genetics Applied to Livestock Production. Belo Horizonte, Brazil: Published by Organizing committee of the 8th World Congress of Genetics Applied to Livestock Production, 2006).

2) Evaluation of individual cows based on milk coagulation properties of the whole lactation

For each cow having at least 3 observations during the 305-day lactation in the data, a coagulation curve was fitted based on its milk coagulation properties, which characterizes the whole lactation of the individual cow.

For doing that, a regression model with random coefficients was used:

$$y_{ijk} = H_k + \sum_{n=1}^{3} b_n P_n(t) + \sum_{n=0}^{3} c_{in} P_n(t) + \varepsilon_{ijk}$$

where

$y_{ijk}$ - $j$-th value of $E_{30}$ or log(R) for cow $i$ of farm $k$ $H_k$ - fixed effect of farm $k$

$P_n(t)$- value of Legendre polynomial of order n at standardized milking day t

$b_n$ ja $c_{in}$ - coefficients of the model, calculated on basis of data

$\varepsilon_{ijk}$ - random error.

In addition to the farm effect the model with random regression coefficients contains 3rd order Legendre polynomial, which is included as a fixed effect factor but also as random effect factor, in order to be able to estimate individual curve of each cow different from the average curve. Legendre polynomials are known for their flexibility in modelling observed data points. In addition, the process of calculation of estimates converges faster when using Legendre polynomials. All statistical analysis was done by using the SAS System® (SAS, Cary, NC, USA).

The results of evaluation of milk coagulation properties of cows under the investigation are disclosed in Table 3. Curd firmness of better cows (n=43) exceeded that of the rest of the cows (n=533) by 31.6% and milk coagulation time of better cows was 22.7% shorter (the average sum of curd firmness of 305-day lactation above 10 606 and average sum of coagulation time below 2206). As many as 7.5% of cows belong to this category. Such cows could be used in generating cow population with modified coagulation properties.

41.7% better curd firmness was achieved when the average sum of curd firmness of the 305 days lactation was above 11 000 (in a group of better cows 8 and in an other group 568 cows) and 28.4% faster coagulation time when the average sum of coagulation time was below 2016. As many as 1.4% cows belong to this category. Such cows with better coagulation properties could be used in a breeding program as bull dams. In the case of a bigger population where the better group consisted of 192 cows (33.3% of the population) and the worse group consisted of 384 cows, the curd firmness of cows in the first group exceeded that of the second group by 27.2% and the coagulation time was shorter by 19.2%.

**Table 1. Curd firmness ($E_{30}$) and coagulation time (R) summarized over 305 lactation days for various selection intensities (i)**

| Trait | Selection intensity (i) | Better cows (1) | | Worse cows (2) | | (1) is greater/ lower than (2) | Better cows (%) |
|---|---|---|---|---|---|---|---|
| | | Average sum of 305 day lactation | n | Average sum of 305 day lactation | n | | |
| $E_{30}$ | 1.098 | 9617.39 | 192 | 7562.870 | 384 | 27.17% greater | 33.3 |
| | 1.887 | 10605.95 | 43 | 8057.453 | 533 | 31.63% greater | 7.5 |
| | 2.547 | 11620.41 | 8* | 8200.203 | 568 | 41.71% greater | 1.4 |
| | 0.453 | 8646.393 | 482** | 6203.374 | 94 | 39.38% greater | 83.7 |
| R | 1.098 | 2422.172 | 192 | 2996.937 | 384 | 19.18% lower | 33.3 |
| | 1.887 | 2205.518 | 43 | 2853.74 | 533 | 22.71% lower | 7.5 |

(continued)

| Trait | Selection intensity (i) | Better cows (1) | | Worse cows (2) | | (1) is greater/ lower than (2) | Better cows (%) |
|---|---|---|---|---|---|---|---|
| | | Average sum of 305 day lactation | n | Average sum of 305 day lactation | n | | |
| | 2.547 | 2016.53 | 8 | 2816.459 | 568 | 28.40% lower | 1.4 |
| | 0.453 | 2675.279 | 482 | 3472.3 | 94 | 22.95% lower | 83.7 |

\* - $E_{30}$ above 11 000

\*\* - $E_{30}$ above 7 000

**EXAMPLE 2**

**Estimation of sires based on coagulation properties of their daughters.**

The study was performed to estimate the heritability of coagulation properties based on daughters of sires and to identify sires with best coagulation properties. The value of heritability was estimated as 0.73 for curd firmness and 0.55 for coagulation time and curd firmness of the best sire was 26.4% greater and coagulation time 20% shorter than in the case of the worst sire. We can conclude that curd firmness and coagulation time are affected by genetic factors and variation between bulls enables to prefer in a selection sires with better curd firmness and/or shorter coagulation time.

Fig. 1 presents the curves of coagulation time averaged over daughters of sires that had at least one significant difference. It is possible to see the differences in a change of coagulation properties between sires during the lactation of their daughters. The variation of coagulation time was rather smaller in the beginning of lactation in comparison with the end of lactation - a quite fast growth trend during the 100 first lactation days was replaced by more stable individual changes of coagulation time. In general it is possible to say that the milk coagulation time had the tendency to increase during the whole lactation.

Figure 2 presents curves of curd firmness which were constructed using same principles as curves of coagulation time. For each test bull selected to the figure there existed at least one significant difference in curd firmness from other selected bulls. In the begginning of lactation there was a decline of firmness of curd that was replaced by growth after 50th days of lactation. Such change was characteristic to all sires, since curves had similar forms.

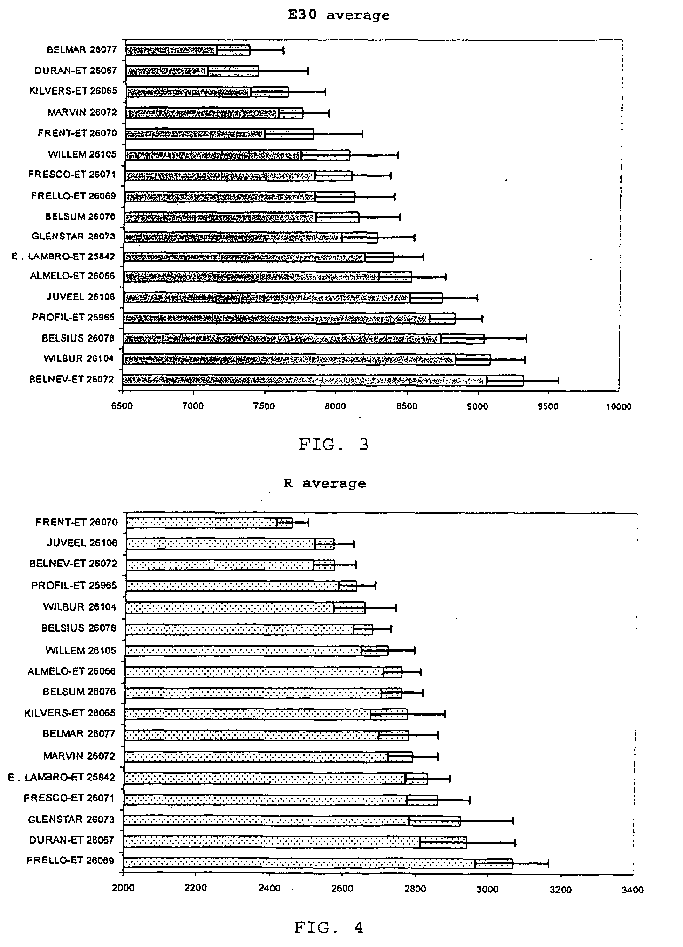

Figure 3 shows that averages S_$E_{30}$ of progeny of different sires are quite different and there also exist statistically different (P<0.05) averages. Ordering sires according to this indicator, the sires with higher average are considered to be better.

Figure 4 presents milk average S_R based on progeny of sires, which is quite variable and there also exist statistically different (P<0.05) averages. Difference between the highest and lowest average reaches almost 600 min. Sires with lower level of the indicator are considered to be better.

On the basis of coagulation curves of daughters, such curves were also calculated for the bulls having at least 10 daughters in the analysis. The curve of coagulation of a bull was calculated as an average of curves of his descendants. In order to compare the bulls, it was necessary to have an indicator as the basis of ordering. Such an indicator was defined as the total value of the coagulation property over 305 milking days. In order to get the value of coagulation property per day, the total value was divided by 305.

On the basis of the total indicator above, heritabilities of coagulation properties were estimated. The bulls were compared by the ANOVA procedure applied to aggregated values of $E_{30}$ and R for daughters of the bulls. The ANOVA procedure comprised as many as 344 daughters of 17 bulls. The comparison of bulls was performed by Bonferroni test. Components of variance and heritability were estimated by the ANOVA method, taking the fixed effect of farm into account. The whole statistical analysis was performed by the SAS System® (SAS, Cary, NC, USA).

**Table 2. Curd firmness (E$_{30}$) and coagulation time (R) of 305-d lactation milk of daughters of sires for various selection intensities (i)**

| Trait | Selection intensity (i) | Better sires (1) | | Worse sires (2) | | (1) is greater/ lower than (2) | Better sires (%) |
|---|---|---|---|---|---|---|---|
| | | Average sum of 305 day lactation of daughters of sires | n | Average sum of 305 day lactation of daughters of sires | n | | |
| E$_{30}$ | 1.667 | 9200.42 | 2 | 8152.13 | 15 | 12.86% greater | 11.8 |
| | 1.181 | 9004.70 | 5 | 7971.609 | 12 | 12.96% greater | 29.4 |
| R | 1.667 | 2509.57 | 2 | 2781.941 | 15 | 9.79% lower | 11.8 |
| | 1.181 | 2574.52 | 5 | 2822.970 | 12 | 8.80% lower | 29.4 |

## EXAMPLE 3

**Identification of genotype of cattle for estimation of coagulation properties during 305-d lactation**

The study was carried out with the aim to identify κ-casein genotype and to find an average daily prognosis of coagulation properties and relationship between the whole lactation coagulation properties and κ-casein genotype.

It was found that by determination of milk coagulation properties of individual cows at least 3 times during the lactation period for every cow it is possible to create a specific coagulation curve, on the basis of which it is possible to estimate the cow by its milk coagulation properties and so the named coagulation curves can be used for the identification of cows with best coagulation properties.

The κ-casein genotype was identified for every cow (DNA was separated from blood) and by using statistical methods, a relationship between the whole lactation coagulation properties and κ-casein genotype was established. All statistical analysis was done by using the SAS System® (SAS, Cary, NC, USA).

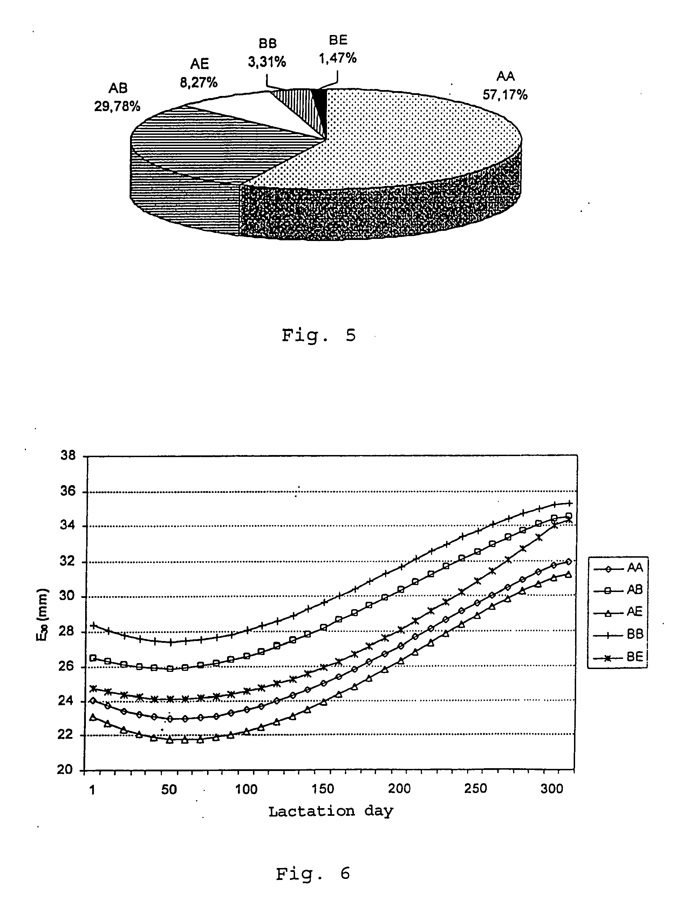

In Figure 6, the average curves of curd firmness are given, averaged over predicted curves of cows with respective genotype.

There is a statistically significant (P<0.001) difference between average curd firmnesses of the whole lactation for different genotypes. One can see the advantage of genotypes with B-allele. Changes during lactation are similar for different genotypes, only the genotype BE has more rapid growth trend in the end of lactation as compared to the others.

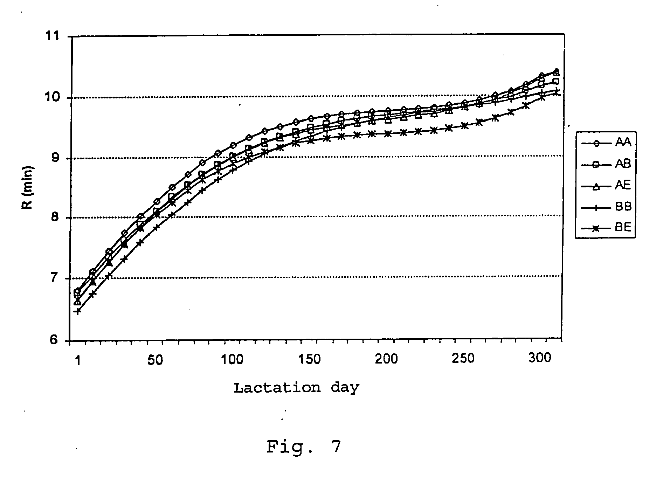

In Figure 7, the lactation curves of milk coagulation time are similarly found as the averages of lactation curves of milk coagulation time of cows with respective κ-casein. There are no differences between whole lactation coagulation times for different genotypes, during lactation all genotypes have similar growth trend.

**Table 3. Estimated components of variance and heritability (h$^2$) of whole lactation milk curd firmness (E$_{30}$) and coagulation time (R)**

| Trait | $\sigma_s^2$ | Standard error | $\sigma_\varepsilon^2$ | Standard error | $h^2$ | Standard error |
|---|---|---|---|---|---|---|
| E$_{30}$ | 250707.9 | 109386.7 | 1121935.0 | 87475.1 | 0.73 | 0.289 |
| R | 15971.7 | 7403.4 | 99477.9 | 7756.1 | 0.55 | 0.254 |
| - variance component caused by bulls ; - variance component caused by random error; $h^2$ - heritability. | | | | | | |

From Table 3, it can be seen that the genetic variation caused by bulls.is quite high as compared to the residual variation of coagulation properties. For this reason the heritability estimates are also high, although they come with rather big standard errors.

Genotype AA is represented most often and the share of A-allele is very high in comparison with B and E allele (Figure 5). Daily firmness of curd differs significantly between genotypes - it was highest for genotype BB and lowest for genotype AE. Daily coagulation time does not differ significantly between genotypes, it was lowest for genotype BE and highest for genotype AA (Table 4).

**Table 4. Frequencies of $\kappa$-casein genotype and allele, and daily prognosis of coagulation properties**

| | Genotype | | | | | Allele | | |
|---|---|---|---|---|---|---|---|---|
| | AA | AB | AE | BB | BE | A | B | E |
| Number of cows | 311 | 162 | 45 | 18 | 8 | | | |
| Frequency | 0.57 | 0.30 | 0.08 | 0.03 | 0.02 | 0.76 | 0.19 | 0.05 |
| Average $E_{30}$ (mm) | 26.07[a] | 29.06[b] | 25.09[a] | 30.43[b] | 27.21[ab] | | | |
| Average R (min) | 9.25 | 9.13 | 9.10 | 8.98 | 8.93 | | | |
| [a,b] Averages with different upper indices differ significantly (P<0.05) | | | | | | | | |

The whole lactation firmness of curd was best for $\kappa$-casein genotype BB and worse for genotype AE and EE. As the milk of cows having $\kappa$-casein E-allele had the worst coagulation properties, a cattle having $\kappa$-casein E-allele is not suggested as sires or bull dams.

The results obtained could be used in a marker selection to evaluate curd firmness of the whole lactation, but $\kappa$-casein genotype did not have impact to whole lactation coagulation time.

The $\kappa$-casein genotypes can not be used for identifying cows that produce milk with modified coagulation properties, since they did not exceed reference values of modified coagulation properties; however, they can be used as an additional marker for identification of cows which produce milk of higher curd firmness ($E_{30}$>30.43; BB-genotype) or lower curd firmness (Eso<26.07; EE-genotype and AE-genotype).

**Claims**

1. A method of identifying of individual cows and bull dams that produce milk with modified coagulation properties and sires whose daughters are expected to produce milk with modified coagulation properties,
   comprising measurement of milk curd firmness and coagulation time of said cows, bull dams and daughters of sires **characterised in that**

   a) milk curd firmness and coagulation time are measured at least 3 times during the whole lactation,
   b) Legendre polynomials are used to estimate the level of these traits within the whole lactation,
   c) individual coagulation curves of curd firmness and coagulation time are obtained,
   d) average levels of said individual coagulation curves of curd firmness and coagulation are compared with reference levels,
   e) cows and bull dams who produce milk with modified coagulation properties during the whole lactation and sires whose daughters are expected to produce milk with modified coagulation properties during the whole lactation are identified if

   the average milk curd firmness of cows is above 34.75 mm and average coagulation time is below 7.2 min,
   the average milk curd firmness of bull dams is above 36 mm and average coagulation time is below 6.6 min and
   the average milk curd firmness of daughters of said sires is above 29.5 mm and coagulation time is below 8.5 min.

2. Method according to the Claim 1 which further comprises determination of $\kappa$-casein genotype of cows, bull dams and daughters of sires and identification that the average curd firmness bigger than $E_{30}$>30.43 corresponds to the $\kappa$-casein genotype BB and the average curd firmness less than $E_{30}$<26.07 corresponds to $\kappa$-casein genotypes EE and AE.

3. Method according to the Claim 2, which further comprises identification that the cows and bull dams and sires whose daughters are expected to produce milk with modified coagulation properties during the whole lactation are not bearing $\kappa$-casein E-allele.

**Patentansprüche**

1. Verfahren zur Identifizierung von Kühen und Zuchtstiermuttern, die Milch mit den modifizierten Coagulationseigenschaften erzeugen sowie von Zuchtbullen, deren Töchter voraussichtlich Milch mit den modifizierten Coagulationseigenschaften erzeugen , die die Messung der Festigkeit des Käsebruches und der Coagulationszeit der Milch von den genannten Kühen, Zuchtstiermütter und Töchter der Zuchtbullen umfasst, **dadurch gekennzeichnet, dass**

   a) die Festigkeit des Käsebruches und die Coagulationszeit der Milch während der ganzen Laktationsperiode mindestens dreimal gemessen werden;
   b) die Werte dieser Indikatoren während der ganzen Laktationsperiode mithilfe von Legendreschen Polynomen bewertet werden;
   c) sich daraus die individuellen Coagulationskurven der Festigkeit des Käsebruches und der Coagulationszeit ergeben;
   d) die durchschnittlichen Werte der erwähnten individuellen Coagulationskurven der Festigkeit des Käsebruches und der Coagulationszeit mit den Bezugswerten verglichen werden;
   e) die Kühe und Zuchtstiermütter, die während der ganzen Laktationsperiode Milch mit den modifizierten Coagulationseigenschaften erzuegen, sowie die Zuchtbullen, deren Töchter während der ganzen Laktationsperiode voraussichtlich Milch mit den modifizierten Coagulationseigenschaften erzeugen identifiziert werden, wenn bei den Kühen die durchschnittliche Festigkeit des Käsebruches der Milch höher als 34,75 mm und die durchschnittliche Coagutationszeit kürzer als 7,2 Min. ist, wenn bei den Zuchtstiermütter die durchschnittliche Festigkeit des Käsebruches der Milch höher als 36 mm und die durchschnittliche Coagulationszeit kürzer als 6,6 Min. ist, und wenn bei den Töchter der genannten Zuchtbullen die durchschnittliche Festigkeit des Käsebruches der Milch höher als 29,5 mm und die Coagulationszeit kürzer als 8,5 Min. ist.

2. Verfahren nach Anspruch 1, wobei zusätzlich noch der Genotyp des $\kappa$-Kaseins der Kühe, Zuchtstiermütter und der Töchter der Zuchtbullen festgestellt wird und bei der es identifiziert wird, dass eine höhere als $E_{30} > 30{,}43$ Festigkeit des Käsebruches der Milch dem Genotyp BB des $\kappa$-Kaseins entspricht und dass eine kleinere als die eben genannte $E_{30} < 26.07$ Festigkeit des Käsebruches der Milch den Genotypen EE und AE des $\kappa$-Kaseins entspricht.

3. Verfahren nach Anspruch 2, wobei es zusätzlich noch identifiziert wird, dass bei den Kühen, Zuchtstiermütter und Zuchtbullen, deren Töchter während der ganzen Laktationsperiode voraussichtlich Milch mit den modifizierten Coagulationseigenschaften erzeugen, das E-Allel des $\kappa$-Kaseins nicht vorhanden ist.


**Revendications**

1. Procédé d'identification de chaque vaches, et mères de taureaux produisant du lait ayant des propriétés de coagulation modifiées et des taureaux dont les filles vont selon les attentes produire du lait avec des propriétés de coagulation modifiées, comprenant le mesurage de la fermeté du lait caillé et du temps de coagulation des vaches, mères de taureaux et des des filles des taureaux susnommés **caractérisé**
**en ce que**

   a) la fermeté du lait caillé et le temps de coagulation sont mesurés au moins 3 fois au cours de la période de lactation entière,
   b) les polynômes de Legendre sont utilisés pour estimer le niveau de ces caractéristiques pendant la période de lactation entière,
   c) les courbes de coagulation individuelle de la fermeté du caillé et le temps de coagulation sont obtenus,
   d) les niveaux moyens de ces courbes de coagulation individuelles de la fermeté du caillé et la coagulation sont comparés avec les niveaux de référence,
   e) les vaches et les mères de taureaux qui produisant du lait ayant des propriétés de coagulation modifiés au cours de la période de lactation entière et les taureaux dont les filles vont selon les attentes produire du lait avec des propriétés de coagulation modifiés au cours de la période de lactation entière sont identifiés au cas ou

   la fermeté moyenne du lait caillé des vaches est supérieure à 34, 75 mm et le temps moyen de coagulation est inférieur à 7,2 min,
   la fermeté moyenne du lait caillé des mères de taureaux est supérieure à 36 mm et le temps moyen de coagulation est inférieur à 6,6 min et
   la fermeté moyenne du lait caillé des filles des taureaux suscités est supérieure à 29,5 mm et le temps de coagulation est inférieur à 8,5 min.

2. Procédé selon la revendication 1 qui comprend en outre la détermination du génotype de caséine K des vaches, des mères à taureaux et des filles des taureaux et l'identification en ce que la fermeté moyenne du caillé supérieure à $E_{30}$> 30,43 correspond au génotype BB de la caséine K, et en ce que la fermeté moyenne du caillé inférieure à $E_{30}$ <26,07 correspond au génotypes EE et AE de la caséine K.

3. Procédé selon la revendication 2, qui comprend en outre l'identification que les vaches et mères de taureaux et les taureaux dont les filles vont selon les attentes produire du lait avec des propriétés de coagulation modifiés au cours de la période de lactation entière ne portent pas d'allèle E de caséine K.

Fig. 1

Fig. 2

EP 2 053 914 B1

## E30 average

FIG. 3

## R average

FIG. 4

Fig. 5

Fig. 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03007705 A

### Non-patent literature cited in the description

- **Ikonen T. et al.** *J. Dairy Sci.,* 2004, vol. 87 (2), 458-467
- **Kubarsepp et al.** *Proc. 22th Nordic Assoc. Agric. Sci. Congr.,* 2003, 90
- **Tyrisevä et al.** *Dairy Sci.,* 2004, vol. 87 (11), 3958-3966
- **Ikonen T.** Department of Animal Science. Academic Diss, 2000
- **Ikonen T. et al.** *Dairy Sci.,* vol. 87 (2), 458-467
- **Ikonen T. et al.** *Agric. Food Sci. Finl.,* 1997, vol. 6, 283-294
- *J. Dairy Sci.,* 1999, vol. 82, 205-214
- *J. Dairy Sci.,* 2004, vol. 87 (2), 458-467
- **Brown, R.J. ; Ernstrom C.A.** Milk clotting enzymes and cheese chemistry. Part I. Fundamentals of Dairy Chemistry. Van Nostrand Reinhold, 1988, 609-633
- Genetic Improvement of Milk Coagulation Properties. **Pärna, E. et al.** Proc. Of the 8th World Congress of Genetics Applied to Livestock Production. 2006
- **Falconer ; Mackay.** Introduction to Quantitative Genetics. Longman, 1996
- **Kinghorn et al.** Use of New Technologies. Twynam Press, 2001